# EUROPÄISCHE PATENTSCHRIFT

(11) **EP 1 333 017 B1**
(45) Veröffentlichungstag und Bekanntmachung des Hinweises auf die Patenterteilung: **21.05.2008**
(21) Anmeldenummer: 03000620.9
(22) Anmeldetag: 15.01.2003
(51) Int. Cl.: C07C 1/20, C07C 41/18, C07C 1/32, C07C 1/24

(54) **Verfahren zur Herstellung von Alkenen**
Process for the production of alkenes
Procédé pour la production d'alcènes

(30) Priorität: 02.02.2002 DE 10204236
(43) Veröffentlichungstag der Anmeldung: 06.08.2003
(73) Patentinhaber: Merck Patent GmbH, 64293 Darmstadt (DE)
(72) Erfinder: Rehm, Daniel, 79539 Lörrach (DE); Dittmann, Marc, 64372 Ober-Ramstadt (DE)

(56) Entgegenhaltungen:
- EP-A- 0 648 722
- WO-A-00/41987
- US-A- 2 332 867
- DATABASE CROSSFIRE BEILSTEIN [Online] Beilstein Institut zur Förderung der Chemischen Wissenschaften, Frankfurt am Main, DE; Database accession no. 293867 XP002259080 & NAKAMURA, S ET AL: JOURNAL OF THE AMERICAN CHEMICAL SOCIETY., Bd. 122, Nr. 34, 2000, Seiten 8120-8130, AMERICAN CHEMICAL SOCIETY, WASHINGTON, DC., US ISSN: 0002-7863
- DATABASE CROSSFIRE BEILSTEIN [Online] Beilstein Institut zur Förderung der Chemischen Wissenschaften, Frankfurt am Main, DE; Database accession no. 470113 XP002259081 & WEISBURGER ET AL: JOURNAL OF ORGANIC CHEMISTRY., Bd. 23, 1958, Seiten 1193-1197, AMERICAN CHEMICAL SOCIETY, WASHINGTON, DC., US ISSN: 0022-3263
- DATABASE CROSSFIRE BEILSTEIN [Online] Beilstein Institut zur Förderung der Chemischen Wissenschaften, Frankfurt am Main, DE; Database accession no. 731567 XP002259082 & LAUS, G ET AL: HETEROCYCLES., Bd. 22, Nr. 2, 1984, Seiten 311-331, ELSEVIER SCIENCE PUBLISHERS B.V. AMSTERDAM., NL ISSN: 0385-5414
- DATABASE CROSSFIRE BEILSTEIN [Online] Beilstein Institut zur Förderung der Chemischen Wissenschaften, Frankfurt am Main, DE; Database accession no. 731560 XP002259083 & MILLS ET AL: JOURNAL OF THE CHEMICAL SOCIETY., 1956, Seiten 4213-4224, CHEMICAL SOCIETY, LONDON, GB ISSN: 0368-1769

## Beschreibung

Die vorliegende Erfindung betrifft ein neuartiges Verfahren zur Herstellung von Alkenen durch Wasserabspaltung aus Alkoholaten bzw. Alkoholen mit mindestens einer CH-Gruppe in α-Stellung zur Alkoholat- bzw. Alkohol-Funktion.

Die Herstellung von Alkenen durch Wasserabspaltung aus Alkoholaten, insbesondere Lithium- oder Grignardalkoholaten, ist prinzipiell bekannt und dient beispielsweise zur Synthese von arylsubstituierten Alkenen, die als flüssigkristalline oder mesogene Substanzen, Pharmawirkstoffe, Pflanzenschutzmittel, Polymere oder Vorstufen zur Herstellung solcher Verbindungen verwendet werden können. Zur Wasserabspaltung wird das Alkoholat, das in α-Position zur Alkoholat-Funktion eine CH-Gruppe aufweist, mit einer Säure umgesetzt.

Die bisher übliche Synthese von arylsubstituierten Alkenen, gemäß der nachstehenden Gleichung 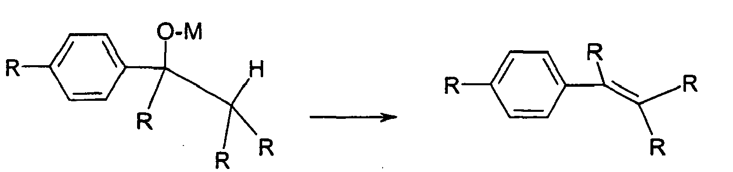 in der R H oder einen Alkylrest bedeutet, wird in einem diskontinuierlichen Verfahren durchgeführt. Hierzu wird die Alkoholatlösung in Gegenwart von Schwefelsäure mehrere Stunden lang erhitzt. Die Reaktionszeit kann in der Regel durch eine höhere Reaktionstemperatur verringert werden. Auf der einen Seite ist jedoch die maximal einstellbare Reaktionstemperatur durch die Siedetemperatur des eingesetzten Lösungsmittels beschränkt, sofern nicht spezielle Druckapparaturen verwendet werden und damit ein deutlich höherer apparativer sowie sicherheitstechnischer Aufwand in Kauf genommen wird. Auf der anderen Seite sind die erhaltenen Alkene je nach Struktur und funktionellen Gruppen unterschiedlich stabil, so dass bei den verhältnismäßig langen Reaktionszeiten im Reaktionsgemisch unerwünschte Folgereaktionen zur Minderung der Produktausbeute und zu einem höheren Aufarbeitungsaufwand führen können. Daher sind insbesondere chemisch anspruchsvolle Alkene durch Wasserabspaltung aus den entsprechenden Alkoholaten nicht oder nur mit geringen Ausbeuten zugänglich.

Aufgabe der vorliegenden Erfindung war es daher, ein Verfahren der eingangs genannten Art bereitzustellen, dass die Synthese der gewünschten Alkene in sehr hohen Ausbeuten bei keiner oder lediglich geringer Nebenproduktbildung ermöglicht, dass gut beherrschbar ist, keinen hohen sicherheitstechnischen Aufwand erfordert und damit auch eine ökonomische Synthese von Alkenen in kommerziellen Maßstäben erlaubt.

Die Aufgabe wird mit einem Verfahren gemäß Anspruch 1 gelöst, wobei die Unteransprüche vorteilhafte Ausgestaltungen des Verfahrens betreffen.

Gegenstand der vorliegenden Erfindung ist somit ein Verfahren zur Herstellung von Alkenen durch Wasserabspaltung aus Alkoholaten bzw. Alkoholen mit mindestens einer CH-Gruppe in α-Stellung zur Alkoholat-bzw. Alkohol-Funktion, bei dem
a) das Alkoholat bzw. der Alkohol in mindestens einem Lösungsmittel vorgelegt und temperiert wird,
b) mindestens eine Säure in mindestens einem Lösungsmittel getrennt vorgelegt und temperiert wird,
c) die Eduktlösungen aus a) und b) getrennt voneinander in mindestens einen temperierten, kontinuierlich betriebenen Durchflussreaktor, der ein oder mehrere Ströhmungsrohre umfasst, geleitet und dort zusammengeführt werden,
d) das Reaktionsgemisch abgeleitet wird und dieses gesammelt, aufgearbeitet und/oder in einer oder mehreren nachgeschalteten Reaktionen weiter umgesetzt wird.

Das erfindungsgemäße Verfahren erlaubt die Synthese von Alkenen aus den entsprechenden Alkoholaten bzw. Alkoholen in hohen bis sehr hohen Ausbeuten. Hierbei handelt es sich um ein kontinuierliches Verfahren, bei dem Volumenströme der Eduktlösungen im Durchflussreaktor zusammengeführt werden und der so erzeugte Volumenstrom des Reaktionsgemisches den temperierten Durchflussreaktor in definierter Weise durchströmt. Das kleine Reaktionsvolumen in Verbindung mit der kontinuierlichen Reaktionsführung erlaubt die Einstellung kurzer Verweilzeiten des Reaktionsgemisches im Durchflussreaktor. Hierdurch wird die Möglichkeit eröffnet, selbst thermisch labile Alkoholate über eine Wasserabspaltung in die entsprechenden Alkene bei hoher Selektivität zu überführen. Ferner werden die erhaltenen Alkene thermisch nur zeitlich gering belastet, so dass Nebenreaktionen weitgehend vermieden werden können. Mit dem erfindungsgemäßen Verfahren sind die Reaktionsparameter, wie Reaktionstemperatur, Druck, Verweilzeit des Reaktionsgemisches sehr exakt einstellbar und damit in Abhängigkeit der Reaktivität und Stabilität der Edukte und Produkte sowie ökonomischer Aspekte sehr gut optimierbar.

Darüber hinaus ist es gegenüber dem bekannten diskontinuierlichen Verfahren von grundlegendem Vorteil, dass das Volumen des Reaktionsgemisches im Durchflussreaktor vergleichsweise klein ist. So sind auch hochreaktive, toxische oder in sonstiger Weise potentiell gefährliche Edukte, Produkte und/oder Lösungsmittel unter sehr gut beherrschbaren Bedingungen ohne zusätzliche aufwändige Sicherheitsvorkehrungen handhabbar. Zudem steht ohne erhöhten Sicherheitsaufwand ein deutlich größerer Wertebereich der Reaktionsparameter zur Verfügung. So sind Wasserabspaltungen bei erhöhten Drücken und/oder bei hohen Temperaturen, insbesondere bei Temperaturen oberhalb der Siedetemperatur des Lösungsmittels, ohne weiteres durchführbar.

Ein weiterer Vorteil ist das günstige Oberflächen- zu Volumen-Verhältnis in einem Durchflussreaktor im Gegensatz zu herkömmlichen Rührwerksapparaturen. Hierdurch kann die Temperatur des Reaktionsmediums schnell und exakt eingestellt werden. Die unkontrollierte Bildung von Bereichen mit erhöhter Temperatur (hot spots), verbunden mit unerwünschten Nebenreaktionen und Sicherheitsproblemen, wird somit weitestgehend vermieden.

Insgesamt sind daher mit dem neuen erfindungsgemäßen Verfahren zum einen Wasserabspaltungen aus Alkoholaten bzw. Alkoholen zugänglich, die mit bekannten diskontinuierlichen Verfahren nicht durchführbar waren, zum anderen sind solche Wasserabspaitungsreaktionen deutlich besser optimierbar. Durch die erzielbare höhere Selektivität der Umsetzung und damit geringere Nebenproduktbildung gestaltet sich die gegebenenfalls anschließende Aufarbeitung einfacher oder kann je nach Art der Folgereaktion gänzlich wegfallen.

Besonders vorteilhaft wird das erfindungsgemäße Verfahren zur Synthese von arylsubstituierten Alkenen eingesetzt, die als flüssigkristalline oder mesogene Substanzen, Pharmawirkstoffe, Pflanzenschutzmittel, Polymere oder Vorstufen zur Herstellung solcher Verbindungen Verwendung finden.

Nachfolgend werden bevorzugte Ausführungsformen und Varianten des erfindungsgemäßen Verfahrens beschrieben.

Vorzugsweise wird als Alkoholat bzw. Alkohol, im folgenden gemeinsam als Alkoholat bezeichnet, eine Verbindung der Formel I verwendet 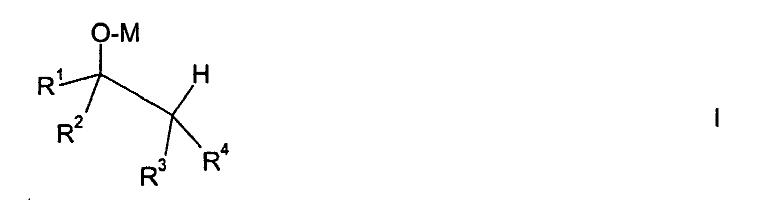 in der
- M: ein Alkalimetall, ein Erdalkalimetallhalogenid oder ein H-Atom bedeutet und
- R¹, R², R³, R⁴: unabhängig voneinander einen ggf. substituierten aliphatischen oder aromatischen Rest, der ein oder mehrere Heteroatome aufweisen kann, bedeuten, wobei ein, zwei oder drei Reste der Gruppe R¹ bis R⁴ auch H bedeuten können, und wobei zwei oder mehr der Reste R¹, R², R³ und/oder R⁴ miteinander verbunden sein können.

Vorzugsweise sind die Reste R¹ und R² und/oder R² und R³ miteinander verbunden, beispielsweise unter Ausbildung eines aliphatischen und/oder aromatischen Rings oder anellierten Ringsystems, der bzw. das auch ein oder mehrere Heteroatome aufweisen kann.

Bevorzugte Bedeutungen von M sind Li, MgCI, MgBr oder Mgl.

Vorzugsweise besitzt R¹ eine Bedeutung gemäß Formel la 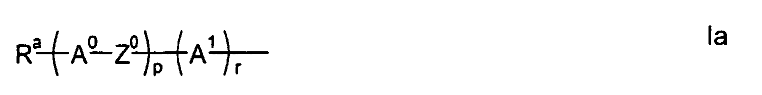 worin
- R^{a}: einen unsubstituierten, einen einfach oder mehrfach durch CN und/oder Halogen substituierten Alkylrest mit 1 bis 15 C-Atomen, wobei in diesen Resten auch eine oder mehrere CH₂-Gruppen durch -O-, -S-, -CH=CH-, -C≡C-, -OC-O-und/oder -O-CO-, und/oder auch eine oder mehrere CH-Gruppen durch N oder P so ersetzt sein können, dass zwei O-Atome nicht direkt miteinander verknüpft sind, oder, falls r und/oder p verschieden von 0 sind, auch H, Halogen, CN, SF₅ oder NCS,
- A⁰, A¹: jeweils unabhängig voneinander
a) einen 1,4-Cyclohexenylen- oder 1,4-Cyclohexylenrest, worin eine oder zwei nicht benachbarte CH₂-Gruppen durch -O- oder -S- ersetzt sein können,
b) einen 1,4-Phenylenrest, worin eine oder zwei CH-Gruppen durch N ersetzt sein können,
c) einen Rest aus der Gruppe Piperidin-1,4-diyl 1,4-Bicyclo[2,2,2]octylen, Phenanthren-2,7-diyl, Naphthalin-2,6-diyl, Decahydronaphthalin-2,6-diyl, und 1,2,3,4-Tetrahydronaphthalin-2,6-diyl,
d) einen bivalenten Rest aus der Gruppe Furan, Pyrrol, Thiophen, Pyrazol, Imidazol, 1,2-Oxazol, 1,3-Oxazol, Thiazol, Pyridin, Pyridazin, Pyrimidin, Pyrazin, 2H-Pyran, 4H-Pyran, Purin, Pteridin, 1H-Azepin, 3H-1,4-Diazepin, Indol, Benzofuran, Benzothiophen, Chinolin, Isochinolin, Phenazin, Phenoxazin, Phenothiazin und 1,4-Benzodiazepin,
   wobei die Reste a), b), c) und d) ein- oder mehrfach durch R^{a}, insbesondere durch Halogen und/oder CN, substituiert sein können,
- Z⁰: -CO-O-, -O-CO-, -CF₂O-, -OCF₂-, -CH₂O-, -OCH₂-, -CH₂CH₂-, -(CH₂)₄-, -C₂F₄-, -CH₂CF₂-, -CF₂CH₂-, -CF=CF-, -CH=CH-, -C≡C- oder eine Einfachbindung,
- p: 0, 1, 2 oder 3 und
- r: 0, 1 oder 2 bedeutet.

Bevorzugte Bedeutungen von R^{a} sind geradkettige oder verzweigte Alkyl- und Alkoxyreste mit 1 bis 8 C-Atomen, die einfach durch -CN und/oder ein- oder mehrfach durch Halogen substituiert sein können.

Bevorzugte Bedeutungen von A⁰ und/oder A¹ sind 1,4-Cyclohexylen, worin eine oder zwei nicht benachbarte CH₂-Gruppen durch -O- ersetzt sein können, 1,4-Phenylen, worin eine oder zwei CH-Gruppen durch N ersetzt sein können, Phenanthren-2,7-diyl-, Naphthalin-2,6-diyl,
Decahydronaphthalin-2,6-diyl, und 1,2,3,4-Tetrahydronaphthalin-2,6-diyl, wobei diese Reste ein- oder mehrfach durch Halogen, insbesondere Fluor und/oder Chlor, CN und/oder gegebenenfalls durch Halogen substituiertes C₁₋₅-Alkyl oder-Alkoxy substituiert sein können

Besonders bevorzugt ist A¹ eine 1,4-Phenylen-Gruppe, die unsubstituiert oder in 2, 3, 5 und/oder 6-Position ein-, zwei-, drei- oder vierfach mit Fluor substituiert ist, womit die erfindungsgemäße Umsetzung nach folgendem Schema verläuft: 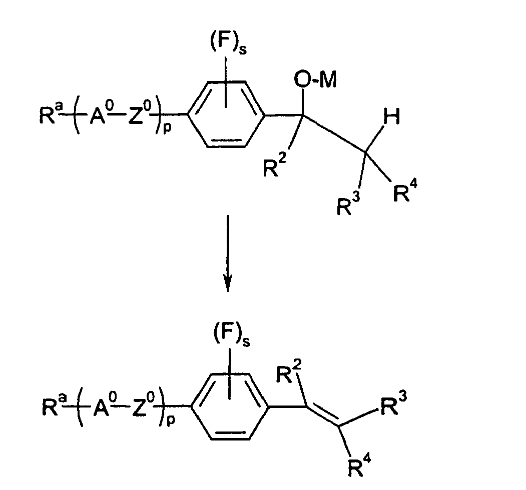 worin R^{a}, A⁰, Z⁰, p, M, R², R³ und R⁴ die vor- und nachstehend angegebenen Bedeutungen aufweisen und s 0, 1, 2, 3 oder 4 bedeutet.

Bevorzugt sind:
- p = r = 0, wobei R^{a} vorzugsweise ein geradkettiger oder verzweigter Alkylrest mit 1 bis 8 C-Atomen ist, der einfach durch -CN und/oder ein- oder mehrfach durch Halogen substituiert sein kann,
- r = 1 und p = 0, 1 oder 2.
R¹ kann auch Bestandteil eines Liganden, beispielsweise eines Cyclopentadienyl-Systems in einem metallorganischen Komplex, sein.

Besonders bevorzugte Gruppen R¹ sind nachfolgend aufgeführt: 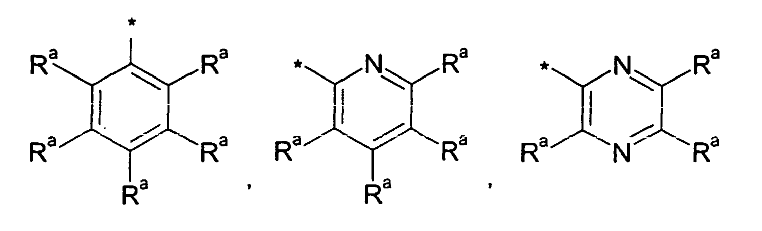 und 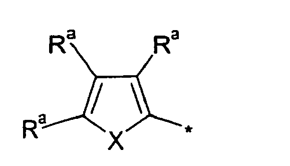 worin X O, NR^{a} oder S bedeutet, R^{a} die angegebene Bedeutung besitzt und * die freie Bindung angibt.

Vor- und nachstehend bedeutet Halogen als Substituent organischer Reste Fluor, Chlor, Brom oder lod, vorzugsweise Fluor oder Chlor, besonders bevorzugt Fluor.

Vor- und nachstehend können mehrfach vorkommende Gruppen und Substituenten, wie beispielsweise A°, Z⁰, A¹, R^{a}, jeweils gleiche oder unterschiedliche Bedeutungen aufweisen.

Vorzugsweise besitzen ein, zwei oder drei Reste der Gruppe R², R³, R⁴ unabhängig voneinander eine Bedeutung gemäß der Formel la und die gegebenenfalls übrigen Reste der Gruppe R², R³, R⁴ bedeuten H.

Besonders bevorzugt ist R⁴ H und R² und/oder R³ besitzen eine von H verschiedene Bedeutung. Ganz besonders bevorzugt sind R² und R³ verschieden von H.

Weiterhin bevorzugt sind R² und R³ derart miteinander verbunden, dass das Alkoholat der Formel I eine Bedeutung gemäß Formel Ib besitzt 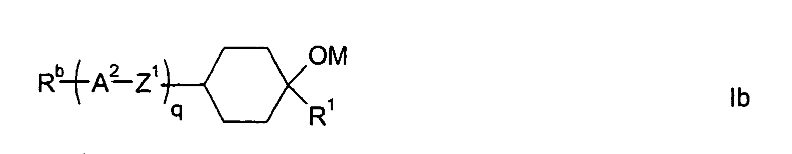 worin
- R^{b}: eine der zu R^{a} angegebenen Bedeutungen besitzt,
- A²: eine der zu A⁰, A¹ angegebenen Bedeutungen besitzt,
- Z¹: eine der zu Z⁰ angegebenen Bedeutungen besitzt,
- q: 0, 1, 2 oder 3 bedeutet und
- R¹ und M: die zuvor und nachfolgend angegebenen Bedeutungen besitzen.

Die Alkoholate der Formel I sind in guten bis sehr guten Ausbeuten durch Addition von metallorganischen Verbindungen an Verbindungen mit einer oder mehreren Carbonylfunktionen erhältlich. Solche Umsetzungen sowie die einzusetzenden Edukte, Lösungsmittel und Reaktionsbedingungen sind dem Fachmann bekannt oder in Abwandlung bekannter Synthesen ohne weiteres zu erhalten.

Unter Einbeziehung der zuvor genannten Synthese der Edukte lässt sich die Umsetzung gemäß des erfinderischen Verfahrens anhand des folgenden Reaktionsschemas darstellen: 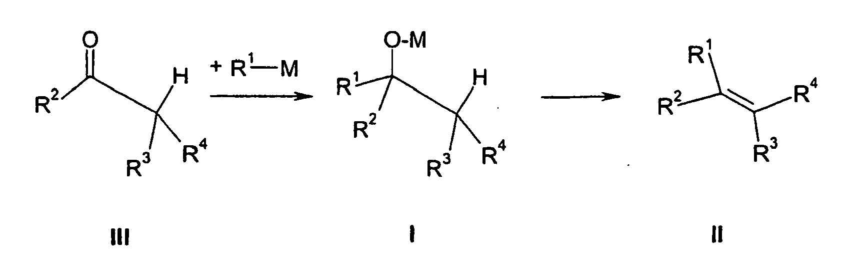 worin M, R¹, R², R³ und R⁴ die angegebenen Bedeutungen besitzen.

Bei Verwendung von metallorganischen Verbindungen R¹-M, insbesondere lithium- oder magnesiumorganischen Verbindungen, mit mindestens einem H-Atom am α-Kohlenstoffatom, beispeilsweise n-Alkyllithium, können auch Ketone ohne Wasserstoffatome an den α-Kohlenstoffatomen, wie beispielsweise Diphenylketon oder Di-tert-butylketon, eingesetzt werden.

Der erste Syntheseschritt, die Addition der metallorganischen Verbindung R¹-M an die Carbonylverbindung der Formel III wird vorteilhaft in einem kontinuierlichen Verfahren durchgeführt. Die Möglichkeit der kontinuierlichen Synthese von Lithiumalkoholaten ist bekannt (DE 198 58 855 A1, DE 198 58 856 A1). Von besonderem Vorteil ist hierbei die Möglichkeit, das kontinuierlich anfallende Alkoholat der Formel I unmittelbar im erfindungsgemäßen Verfahren weiter umzusetzen. Auf eine Aufarbeitung und Isolierung des Alkoholats aus der Reaktionslösung der ersten Stufe wird in einer besonders bevorzugten Variante verzichtet. Damit kann ausgehend von einer metallorganischen Verbindung R¹-M und einer Carbonylverbindung der Formel III das Alken der Formel II in einem über zwei Stufen kontinuierlich durchzuführenden Verfahren erhalten werden.

Nachfolgend werden bevorzugte Reaktionsbedingungen des erfindungsgemäßen Verfahrens aufgeführt.

Bevorzugt wird als Säure Essigsäure, insbesondere ein Gemisch aus Essigsäure mit mindestens einer starken Säure, verwendet. Hierbei bevorzugte starke Säuren sind Salzsäure, Schwefelsäure, Phosphorsäure und/oder Trifluormethansulfonsäure. Vorteilhaft wird das Säuregemisch in einem Lösungsmittel oder Lösungsmittelgemisch temperiert vorgelegt und dem Durchflussreaktor zugeführt.

Die Säure bzw. die Säuren werden vorteilhaft im molaren Überschuss, vorzugsweise im Bereich von 100 Mol% bis 1000 Mol%, besonders bevorzugt von 100 Mol% bis 600 Mol%, insbesondere von 150 Mol% bis 450 Mol%, bezogen auf das eingesetzte Alkoholat zugeführt. Vorteilhaft wird die Gesamtkonzentration der Säuren so gewählt, dass unter den gegebenen Reaktionsbedingungen Feststoffe im Durchflussreaktor nicht oder nur zu solch einem geringen Maße entstehen, dass Verstopfungen von Kanälen des Durchflussreaktors nicht auftreten. Wird ein Gemisch aus Essigsäure mit mindestens einer starken Säure eingesetzt, so beträgt das molare Verhältnis der Essigsäure zu der Gesamtheit der starken Säuren vorzugsweise 1 : 5 bis 5 : 1, besonders bevorzugt 1 : 2 bis 2 : 1, insbesondere in etwa äquimolar.

Bevorzugte Lösungmittel oder Lösungsmittelgemische für das Alkoholat als Edukt und für die Säure bzw. das Säuregemisch sind unter den jeweiligen Reaktionsbedingungen im wesentlichen inerte organische Lösungsmittel, besonders bevorzugt Alkane, Aromaten und Ether, wie beispielsweise Pentan, Hexan, Heptan, Octan, Benzol, Toluol, Xylole, Diethylether, Tetrahydrofuran, 1,4-Dioxan und deren Gemische. Die zu wählende Konzentration der Eduktlösungen ist an sich unkritisch und daher über einen weiten Bereich wählbar.

Die Eduktlösungen werden im Durchflussreaktor vorzugsweise bei einem Druck von über 1 bar, besonders bevorzugt von über 2 bar, insbesondere bei 4 bis 5 bar, zusammengeführt. Bevorzugte, im Durchflussreaktor einzustellende Reaktionstemperaturen liegen im Bereich von 50°C bis 180°C, besonders bevorzugt von 70°C bis 150°C, insbesondere bei 90°C bis 130°C. Ganz besonders bevorzugt wird das Reaktionsgemisch im Durchflussreaktor auf eine Temperatur oberhalb der bei Normaldruck bestimmten Siedetemperatur des Reaktionsgemisches temperiert.

Die Zusammenführung der Eduktlösungen erfolgt vorteilhaft unter Einsatz eines oder mehrerer hintereinander oder parallel geschalteter Y-Stücke, T-Stücke und/oder Statikmischer, an die sich ein oder mehrere parallel geschaltete Strömungsrohre zur Aufnahme und Weiterleitung des Reaktionsgemisches anschließen. Grundsätzlich ist die Kombination eines oder mehrerer Y- oder T-Stücke mit einem oder mehreren Strömungsrohren ausreichend. Zur Erzielung einer besonders hohen Mischungsgüte ist es von Vorteil, zwischen dem oder den Y- oder T-Stücken und dem Strömungsrohr ein oder mehrere Statikmischer fluidisch zwischen zu schalten. Die Y-Stücke, T-Stücke und/oder Statikmischer sowie die Strömungsrohre werden zur Einstellung der gewünschten Reaktionstemperatur unter Einsatz bekannter Maßnahmen, wie beispielsweise eines Temperierbades oder elektrischer Heizelemente, temperiert und sind Bestandteil des Durchflussreaktors. Des weiteren sind Mikroreaktoren als Durchflussreaktor in dem erfindungsgemäßen Verfahren einsetzbar. Geeignete Statikmischer, wie beispielsweise Sulzer-, Kenics-, Kugel- und Mikromischer, sowie Mikroreaktoren und deren Einsatz sind dem Fachmann bekannt, beispielsweise aus ULLMANN'S ENCYCLOPEDIA OF INDUSTRIAL CHEMISTRY, Release 2001, 6th Edition sowie der darin zitierten Literatur. Um eine schnelle und effektive Mischung zu erzielen, weisen die Leitungs- oder Kanalstrukturen, insbesondere der Bereiche des Durchflussreaktors, in denen die Eduktlösungen zusammengeführt werden, vorteilhaft Innenabmessungen in zumindest einer Dimension von ≤ 10 mm, bevorzugt ≤ 3 mm, besonders bevorzugt ≤ 1 mm auf. Zumindest die durchströmten Bereiche des Durchflussreaktors bestehen vorteilhaft aus gegenüber den eingesetzten Reagenzien inerten Materialien, wie beispielsweise Edelstahl, Titan, Glas oder Quarzglas. Zur Kontrolle und damit Steuerung und Optimierung der Wasserabspaltung sind vorteilhaft im Durchflussreaktor sowie gegebenenfalls der nachgeschalteten Verweilstrecke und/oder Abkühlstrecke Mittel zur Kontrolle der Prozessparameter, wie Druck, Temperatur, Volumenfluss und Konzentrationen, integriert. Vorteilhaft werden die so ermittelten Prozessparameter von einer Steuereinheit erfasst, die im Abgleich mit vorgegebenen oder berechneten Sollwerten die Verfahrensführung, beispielsweise durch Ansteuerung von Ventilen, Pumpen und Thermostaten, vornimmt.

Die Zuführung der Eduktlösungen und der Aufbau des im Durchflussreaktors gewünschten Drucks erfolgt mit Hilfe dem Fachmann bekannter Mittel, wie beispielsweise Zahnradpumpen.

Von besonderem Vorteil des erfindungsgemäßen Verfahrens ist die Optimierbarkeit der Wasserabspaltung über eine Einstellung der Verweilzeit des Reaktionsgemisches im Durchflussreaktor. Die Einstellung kann über die Dimensionierung des Durchflussreaktors, insbesondere über die Wahl des Volumens der vom Reaktionsgemisch durchströmten Bereiche und bei gleichem Querschnitt dieser Bereiche über deren Länge erfolgen. Es ist von Vorteil, dem Durchflussreaktor eine temperierte Verweilstrecke nachzuschalten, in die vor der Durchführung des Schritts d) das Reaktionsgemisch aus dem Durchflussreaktor geleitet wird. Über die Wahl der Länge der Verweilstrecke, die vorteilhaft auf im wesentlichen die gleiche Temperatur wie der Durchflussreaktor temperiert ist, ist die Verweilzeit des Reaktionsgemisches konstruktiv einfacher einzustellen als durch konstruktive Änderungen des Durchflussreaktors selbst. Im einfachsten Fall ist die Verweilstrecke eine sich unmittelbar an den Durchflussreaktor anschließende Rohrleitung, vorteilhaft mit im wesentlichen gleichem Durchmesser wie die Reaktionsstrecke im Durchflussreaktor.

Bei gegebener konstruktiver Ausgestaltung des Durchflussreaktors und der gegebenenfalls vorhandenen Verweilstrecke ist die Verweilzeit des Reaktionsgemisches in diesen über die Volumenflüsse der Eduktlösungen einstellbar. Vorteilhafte Verweilzeiten betragen 0,1 bis 600 Sekunden, besonders bevorzugt 0,5 bis 100 Sekunden, insbesondere 1 bis 20 Sekunden.

Vorteilhaft schließt sich an den Durchflussreaktor und der ggf. vorhandenen Verweilstrecke eine niedriger temperierte Abkühlstrecke zum Abkühlen des Reaktionsgemisches an. Mit dem Abkühlen des Reaktionsgemisches können im Anschluss an die Passage durch den Durchflussreaktor und die Verweilstrecke und damit zeitlich exakt nach der eingestellten Verweilzeit des Reaktionsgemisches unerwünschte Neben- oder Folgereaktionen wirkungsvoll unterbunden werden.

Die aus dem Durchflussreaktor abgeleitete Reaktionsmischung, vorzugsweise im Anschluss an die Verweilstrecke oder die Abkühlstrecke, wird vorteilhaft auf Umgebungsdruck entspannt und in einen Vorratsbehälter geleitet oder unmittelbar aufgearbeitet und/oder in weiteren Syntheseschritten eingesetzt.

Die folgenden Ausführungsbeispiele sollen die Erfindung erläutern, ohne sie zu begrenzen. Vor- und nachstehend bedeuten Prozentangaben Gewichtsprozent. Alle Temperaturen sind in Grad Celsius angegeben.

### Versuchsaufbau

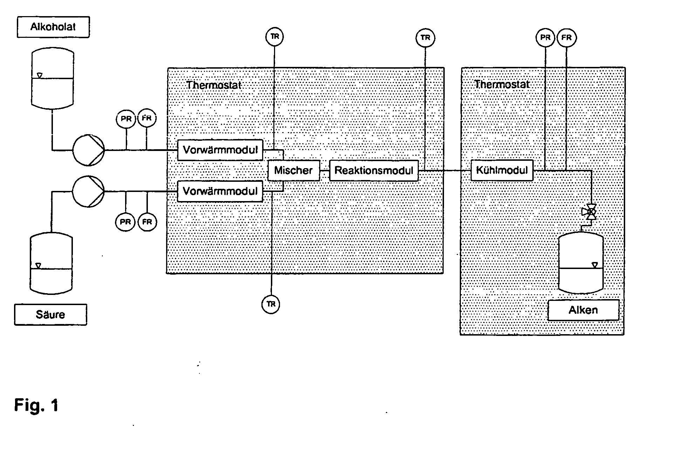

In Fig. 1 ist der Versuchsaufbau schematisch dargestellt. Von den Vorratsbehältern für die beiden Edukte (Alkoholat, Säure) führen jeweils eine Zahnradpumpe aufweisende Leitungen zu jeweils einem Wärmetauscher, hier als Vorwärmmodul bezeichnet, zur Temperierung der Edukte. Die beiden Vorwärmmodule sind fluidisch mit einem 180° T-Stück, hier als Mischer bezeichnet, verbunden, an das sich ein Strömungsrohr und eine Verweilstrecke, hier gemeinsam als Reaktionsmodul bezeichnet, anschließen. Der gesamte Durchflussreaktor, bestehend aus den beiden Vorwärmmodulen, dem Mischer und dem Reaktionsmodul, wird von einem Thermostaten temperiert. Das Reaktionsmodul ist fluidisch mit einer Abkühlstrecke (Kühlmodul) verbunden, die über ein Druckventil zu einem Vorratsbehälter für das Produktgemisch (Alken) führt. Das Kühlmodul und der Vorratsbehälter werden von einem zweiten Thermostaten temperiert. Als Thermostaten werden Temperierbäder verwendet. In den beiden Eduktleitungen sowie in der Produktleitung im Anschluss an das Kühlmodul sind jeweils Durchflussmesser (FR) und Druckmesser (PR) angebracht. Die Temperatur der Eduktlösungen vor dem Eintritt in den Mischer und der Reaktionslösung im Ausleitungsbereich des Reaktionsmoduls wird durch Temperaturfühler (TR) erfasst.

Die Vorwärmmodule, das Reaktions- und das Kühlmodul bestehen aus VA-Rohr mit einem Innendurchmesser. Die beiden Rohre, einer Länge von jeweils 1 m, des Vorwärmmoduls sind mit dem Rohr des Reaktionsmoduls (Länge 6 m, einschließlich Verweilstrecke) über ein 180° T-Stück miteinander verbunden.

Die Temperaturen in den Thermostaten und der Druck werden manuell vorgegeben. Die Einstellung der Flüsse sowie auch eine kontinuierliche Erfassung der gemessenen Temperaturen, Drücke, Massendurchflüsse und Dichten der Reagentien erfolgt über einen als Steuereinheit dienenden Computer.

### Versuchsdurchführung

Es wurde die Synthese der Cyclohexen-Verbindung 2 untersucht, die ein wichtiges Vorprodukt bei der Synthese von Flüssigkristallen darstellt. Die Verbindung 2 wird durch Wasserabspaltung aus dem entsprechenden Cyclohexanolat-Derivat der Formel 1 unter Einsatz eines Essigsäure/ Schwefelsäure-Gemisches und unter Verwendung der zuvor beschriebenen Anlage gemäß foigender Reaktionsgleichung erhalten:

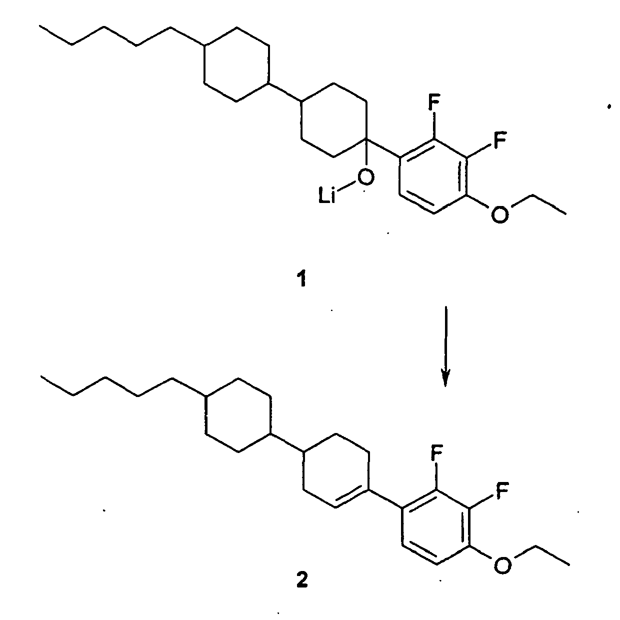

Die verwendeten Eduktlösungen in Tetrahydrofuran des Alkoholat (0,366 mol/l) und des Essigsäure/ Schwefelsäure-Gemisches, jeweils einer Säure-Konzentration von 0,620 mol/l, wurden nach Temperierung in den Vorwärmmodulen jeweils mit einem Massenfluss von 1,00 kg/h, entsprechend einem Durchsatz des Alkoholats von 0,457 mol/h, der Essigsäure und der Schwefelsäure von jeweils 0,732 mol/h, im Mischer zusammengeführt. Über das erste Temperierbad wurde im Reaktionsmodul nacheinander eine Temperatur von a) 95°C, b) 105°C, c) 115°C und d) 125°C bei jeweils einem Druck von 4,5 bar eingestellt. Die Verweilzeit des Reaktionsgemisches im Reaktionsmodul lag bei etwa 4 Sekunden. Die Reaktionslösung wurde im Kühlmodul auf eine Temperatur von 30°C gekühlt und nach Entspannung über das Druckventil in den Vorratsbehälter geleitet.

Zur Untersuchung des Produktgemisches wurden Proben dieses Gemisches mit Wasser ausgeschüttelt und die organische Phase wiederholt mit wässriger Natriumhydrogencarbonatlösung ausgeschüttelt. Der Umsatz wurde aus dem gaschromatographisch bestimmten Gehalt an dem Cyclohexen-Derivat 2 im Vergleich zu nicht umgesetzten Alkoholat 1 berechnet.

| Reaktionstemperatur | Umsatz |
|---|---|
| 95°C | 72,63 % |
| 105°C | 84,17 % |
| 115°C | 94,17 % |
| 125°C | 100 % |

### Synthese des Alkoholats

Die Synthese des Alkoholats 1 aus dem Cyclohexanon-Derivat 5 und der Phenyllithium-Verbindung 4, erhältlich durch Lithiierung von 2,3-Difluorethoxybenzol 3, erfolgt gemäß folgendem Reaktionsschema:

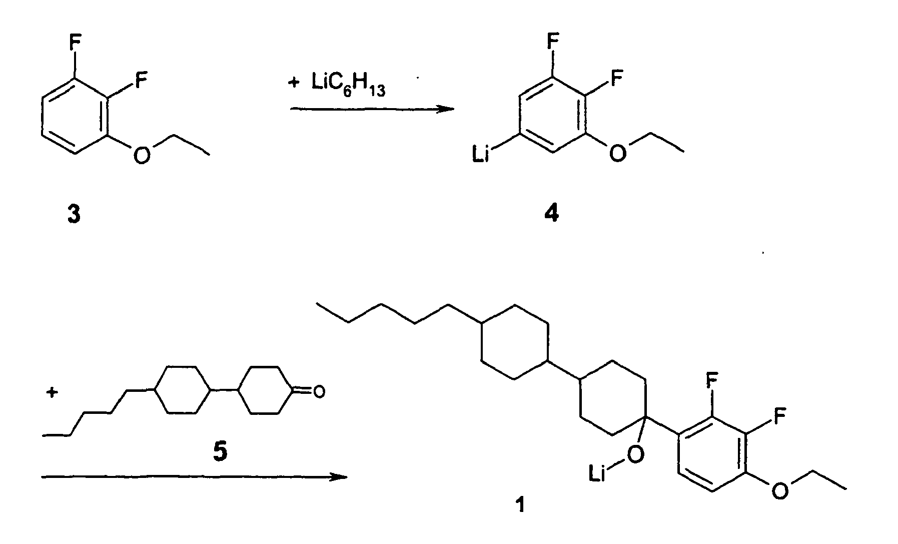

Die Umsetzung erfolgt in einer Apparatur wie sie in der DE 198 58 856 A1 abgebildet und beschrieben ist sowie entsprechend dem darin erläuterten Verfahren. Zur Herstellung der Phenyllithium-Verbindung 4 werden eine Lösung von 2,3-Difluorethoxybenzol 3 (1,0 mol/l) und 2 % 2,2,6,6-Tetramethylpiperidin in Tetrahydrofuran und eine Lösung von Hexyllithium (2,5 mol/l) in Hexan mit einem Massedurchfluss von jeweils 2 mol/h kontinuierlich zusammengeführt und zur Umsetzung gebracht. Die so erhaltene Produktlösung wird kontinuierlich weiter mit einer Lösung des Cyclohexan-Derivats 5 (1 mol/l) in Tetrahydrofuran bei einem Massedurchfluss ebenfalls von 2 mol/h zusammengeführt. Mittels eines Kryostaten wurden Reaktionstemperaturen von -30 bis -35°C eingestellt. Mit diesem zweistufigen kontinuierlichen Verfahren wird ein Umsatz von ≥ 90 % erzielt.

Das erfindungsgemäße Verfahren ist mit einer Vielzahl von Alkoholaten durchführbar. Zur Illustration sind nachfolgend metallorganische Verbindungen (Tabelle 1) und Carbonylverbindungen (Tabelle 2) aufgeführt, aus denen jeweils durch Umsetzung miteinander gemäß obigem Beispiel eine Vielzahl von Alkoholaten zugänglich sind, die in der erfindungsgemäßen Wasserabspaltung zu den gewünschten Alkenen führen.

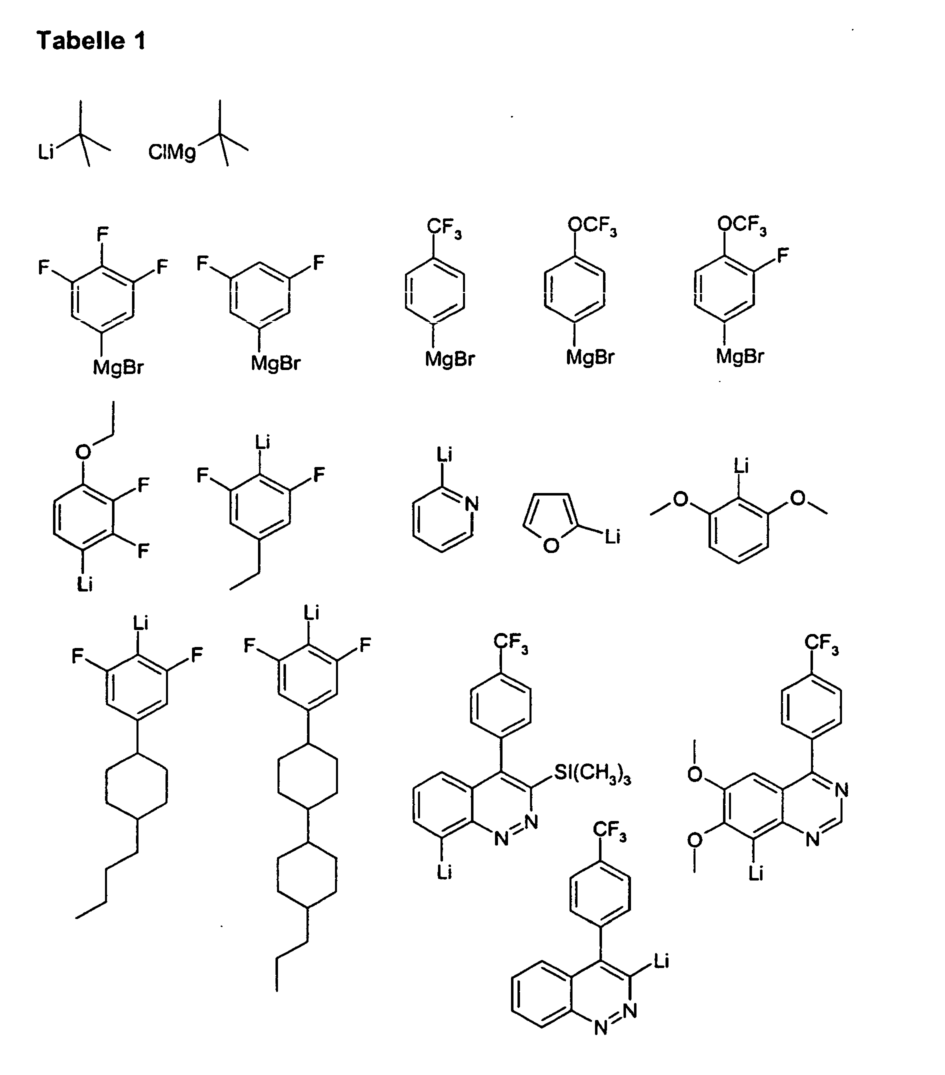

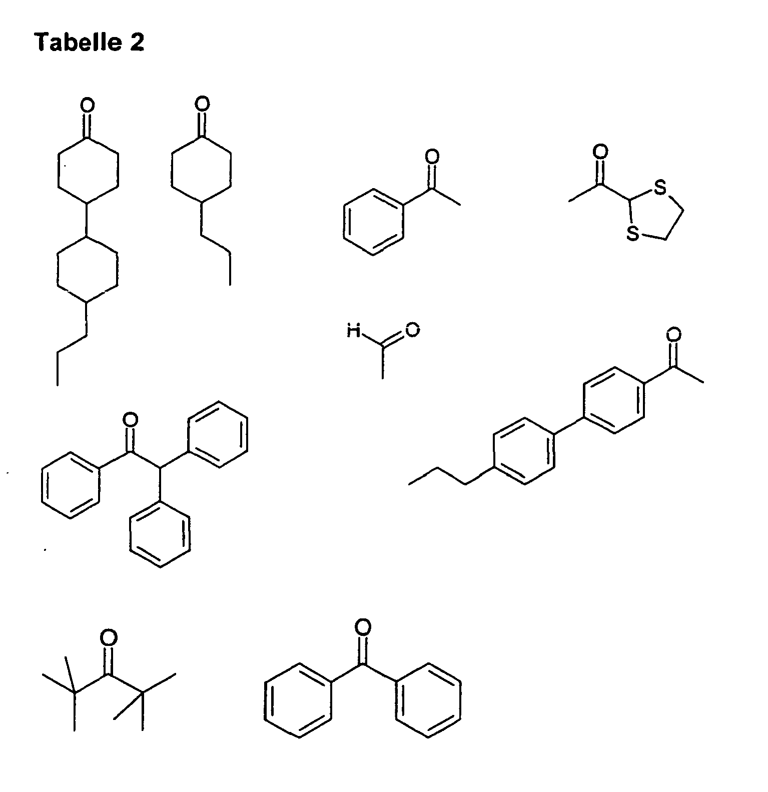

### Synthese von Liganden metallorganischer Verbindungen

Nachfolgendes Syntheseschema veranschaulicht die Synthese von 1,1-Bis(propen-2-yl)ferrocen 7 aus dem Bisalkoholat-Komplex 6 mit Hilfe des erfindungsgemäßen Verfahrens, wobei der Bisalkoholat-Komplex 6 aus Bislithiumferrocen 5 und Aceton zugänglich ist:

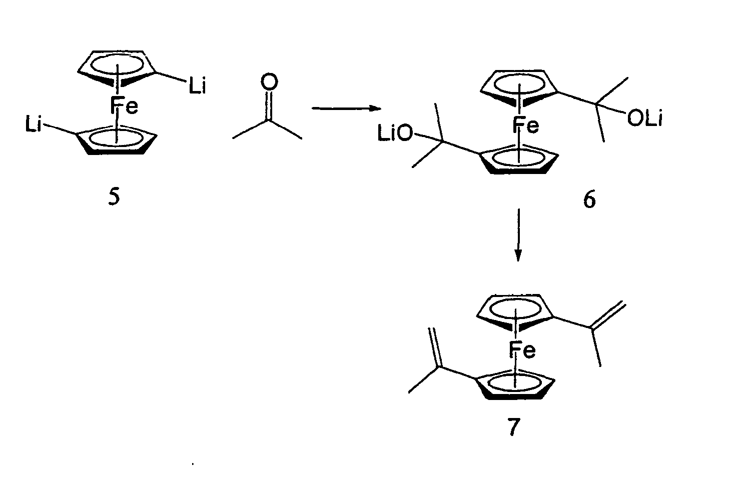

## Patentansprüche

1. Verfahren zur Herstellung von Alkenen durch Wasserabspaltung aus Alkoholaten bzw. Alkoholen mit mindestens einer CH-Gruppe in α-Stellung zur Alkoholat- bzw. Alkohol-Funktion, **dadurch gekennzeichnet, dass**
a) das Alkoholat bzw. der Alkohol in mindestens einem Lösungsmittel vorgelegt und temperiert wird,
b) mindestens eine Säure in mindestens einem Lösungsmittel getrennt vorgelegt und temperiert wird,
c) die Eduktlösungen aus a) und b) getrennt voneinander in mindestens einen temperierten, kontinuierlich betriebenen Durchflussreaktor, der ein oder mehrere Strömungsrohre umfasst, geleitet und dort zusammengeführt werden,
d) das Reaktionsgemisch abgeleitet wird und dieses gesammelt, aufgearbeitet und/oder in einer oder mehreren nachgeschalteten Reaktionen weiter umgesetzt wird.

2. Verfahren nach Anspruch 1, **dadurch gekennzeichnet, dass** als Alkoholat bzw. Alkohol eine Verbindung der Formel I 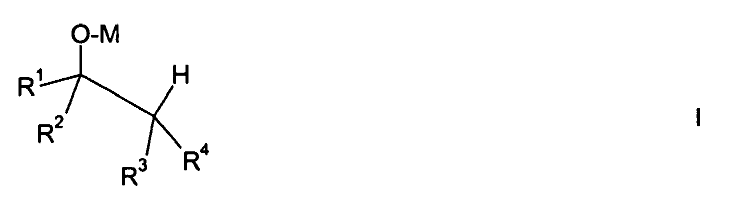 verwendet wird, in der
M ein Alkalimetall, ein Erdalkalimetallhalogenid oder ein H-Atom bedeutet und
R¹, R², R³, R⁴ unabhängig voneinander einen ggf. substituierten aliphatischen oder aromatischen Rest, der ein oder mehrere Heteroatome aufweisen kann, bedeuten, wobei ein, zwei oder drei Reste der Gruppe R¹ bis R⁴ auch H bedeuten können, und wobei zwei oder mehr der Reste R¹, R², R³ und/oder R⁴ miteinander verbunden sein können.

3. Verfahren nach Anspruch 2, **dadurch gekennzeichnet, dass**
M Li, MgCl, MgBr oder Mgl bedeutet.

4. Verfahren nach Anspruch 2 oder 3, **dadurch gekennzeichnet, dass** R¹ eine Bedeutung der Formel la aufweist 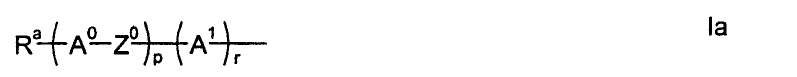 worin
R^{a} einen unsubstituierten, einen einfach oder mehrfach durch CN und/oder Halogen substituierten Alkylrest mit 1 bis 15 C-Atomen, wobei in diesen Resten auch eine oder mehrere CH₂-Gruppen durch -O-, -S-, -CH=CH-, -C≡C-, -OC-O- und/oder -O-CO-, und/oder auch eine oder mehrere CH-Gruppen durch N oder P so ersetzt sein können, dass zwei O-Atome nicht direkt miteinander verknüpft sind, oder, falls r und/oder p verschieden von 0 sind, auch H, Halogen, CN, SF₅ oder NCS,
A⁰, A¹ jeweils unabhängig voneinander
a) einen 1,4-Cyclohexenylen- oder 1,4-Cyclohexylenrest, worin eine oder zwei nicht benachbarte CH₂-Gruppen durch -O- oder -S- ersetzt sein können,
b) einen 1,4-Phenylenrest, worin eine oder zwei CH-Gruppen durch N ersetzt sein können,
c) einen Rest aus der Gruppe Piperidin-1,4-diyl, 1,4-Bicyclo[2,2,2]octylen, Phenanthren-2,7-diyl, Naphthalin-2,6-diyl, Decahydronaphthalin-2,6-diyl, und 1,2,3,4-Tetrahydronaphthalin-2,6-diyl,
d) einen bivalenten Rest aus der Gruppe Furan, Pyrrol, Thiophen, Pyrazol, imidazol, 1,2-Oxazol, 1,3-Oxazol, Thiazol, Pyridin, Pyridazin, Pyrimidin, Pyrazin, 2H-Pyran, 4H-Pyran, Purin, Pteridin, 1H-Azepin, 3H-1,4-Diazepin, Indol, Benzofuran, Benzothiophen, Chinolin, Isochinolin, Phenazin, Phenoxazin, Phenothiazin und 1,4-Benzodiazepin,
wobei die Reste a), b), c) und d) ein oder mehrfach durch R^{a}, insbesondere durch Halogen und/oder CN, substituiert sein können,
Z⁰ -CO-O-, -O-CO-, -CF₂O-, -OCF₂-, -CH₂O-, -OCH₂-, -CH₂CH₂-, -(CH₂)₄-, -C₂F₄-, -CH₂CF₂-, -CF₂CH₂-, -CF=CF-, -CH=CH-, -C≡C- oder eine Einfachbindung,
p 0, 1, 2 oder 3 und
r 0, 1 oder 2 bedeutet.

5. Verfahren nach einem der vorherigen Ansprüche, **dadurch gekennzeichnet, dass** ein, zwei oder drei Reste der Gruppe R², R³, R⁴ unabhängig voneinander eine Bedeutung gemäß der Formel la nach Anspruch 4 besitzen und die gegebenenfalls übrigen Reste der Gruppe R², R³, R⁴ H bedeuten.

6. Verfahren nach einem der vorherigen Ansprüche, **dadurch gekennzeichnet, dass** als Säure ein Gemisch aus Essigsäure mit mindestens einer starken Säure verwendet wird.

7. Verfahren nach Anspruch 6, **dadurch gekennzeichnet, dass** als starke Säure Salzsäure, Schwefelsäure, Phosphorsäure und/oder Trifluormethansulfonsäure verwendet wird.

8. Verfahren nach einem der vorherigen Ansprüche, **dadurch gekennzeichnet, dass** die Eduktlösungen im Durchflussreaktor bei einem Druck von über 1 bar zusammengeführt werden.

9. Verfahren nach einem der vorherigen Ansprüche, **dadurch gekennzeichnet, dass** die Eduktlösungen im Durchflussreaktor bei einer Temperatur von 50°C bis 180°C zusammengeführt werden.

10. Verfahren nach einem der vorherigen Ansprüche, **dadurch gekennzeichnet, dass** der Durchflussreaktor auf eine Temperatur oberhalb der bei Normaldruck bestimmten Siedetemperatur des Reaktionsgemisches temperiert wird.

11. Verfahren nach einem der vorherigen Ansprüche, **dadurch gekennzeichnet, dass** vor der Durchführung des Schritts d) das Reaktionsgemisch aus dem Durchflussreaktor in eine diesem nachgeschaltete, temperierte Verweilstrecke geleitet wird.

12. Verfahren nach einem der vorherigen Ansprüche, **dadurch gekennzeichnet, dass** die Volumenflüsse der Eduktlösungen derart gewählt werden, dass die Verweilzeit des Reaktionsgemisches im Durchflussreaktor und in der ggf. vorhandenen Verweilstrecke 0,1 bis 600 Sekunden beträgt.

13. Verfahren nach einem der vorherigen Ansprüche, **dadurch gekennzeichnet, dass** vor der Durchführung des Schritts d) das Reaktionsgemisch aus dem Durchflussreaktor und der ggf. vorhandenen Verweilstrecke in eine temperierte Abkühlstrecke zum Abkühlen des Reaktionsgemisches geleitet wird.

## Claims

1. Process for the preparation of alkenes by elimination of water from alcoholates or alcohols having at least one CH group in the α-position to the alcoholate or alcohol function, **characterised in that**
a) the alcoholate or alcohol is initially introduced in at least one solvent and brought to temperature,
b) at least one acid is initially introduced separately in at least one solvent and brought to temperature,
c) the starting-material solutions from a) and b) are fed separately from one another into at least one temperature-controlled, continuously operated flow reactor which includes one or more flow tubes and combined there,
d) the reaction mixture is withdrawn and collected, worked up and/or reacted further in one or more downstream reactions.

2. Process according to Claim 1, **characterised in that** the alcoholate or alcohol used is a compound of the formula I 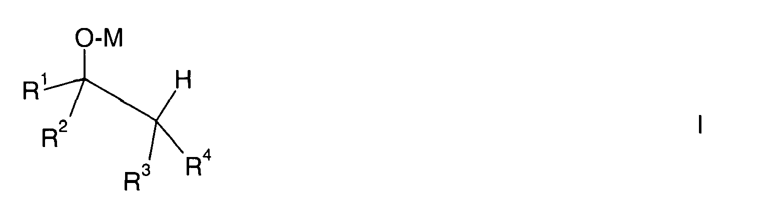 in which
M denotes an alkali metal, an alkaline-earth metal halide or an H atom and
R¹, R², R³, R⁴, independently of one another, denote an optionally substituted aliphatic or aromatic radical, which may have one or more heteroatoms, where one, two or three radicals from the group R¹ to R⁴ may also denote H, and where two or more of the radicals R¹, R², R³ and/or R⁴ may be bonded to one another.

3. Process according to Claim 2, **characterised in that**
M denotes Li, MgCl, MgBr or Mgl.

4. Process according to Claim 2 or 3, **characterised in that** R¹ has a meaning as for formula la 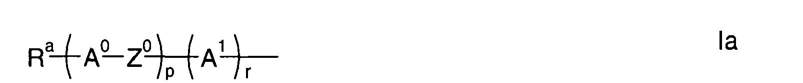 in which
R^{a} denotes an alkyl radical having 1 to 15 C atoms which is unsubstituted, mono- or polysubstituted by CN and/or halogen, where, in addition, one or more CH₂ groups in these radicals may also be replaced by -O-, -S-, -CH=CH-, -C≡C-, -OC-O-and/or -O-CO- and/or, in addition, one or more CH groups may be replaced by N or P in such a way that two O atoms are not linked directly to one another, or, if r and/or p are different from 0, also H, halogen, CN, SF₅ or NCS,
A⁰, A¹ each, independently of one another, denote
a) a 1,4-cyclohexenylene or 1,4-cyclohexylene radical, in which one or two non-adjacent CH₂ groups may be replaced by -O- or -S-,
b) a 1,4-phenylene radical, in which one or two CH groups may be replaced by N,
c) a radical from the group piperidine-1,4-diyl, 1,4-bicyclo-[2,2,2]octylene, phenanthrene-2,7-diyl, naphthalene-2,6-diyl, decahydronaphthalene-2,6-diyl and 1,2,3,4-tetrahydronaphthalene-2,6-diyl,
d) a divalent radical from the group furan, pyrrole, thiophene, pyrazole, imidazole, 1,2-oxazole, 1,3-oxazole, thiazole, pyridine, pyridazine, pyrimidine, pyrazine, 2H-pyran, 4H-pyran, purine, pteridine, 1H-azepine, 3H-1,4-diazepine, indole, benzofuran, benzothiophene, quinoline, isoquinoline, phenazine, phenoxazine, phenothiazine and 1,4-benzodiazepine,
where the radicals a), b), c) and d) may be mono- or polysubstituted by R^{a}, in particular by halogen and/or CN,
Z° denotes -CO-O-, -O-CO-, -CF₂O-, -OCF₂-, -CH₂O-, -OCH₂-, -CH₂CH₂-, -(CH₂)₄-, -C₂F₄-, -CH₂CF₂-, -CF₂CH₂-, -CF=CF-, -CH=CH-, -C≡C- or a single bond,
p denotes 0, 1, 2 or 3 and
r denotes 0, 1 or 2.

5. Process according to one of the preceding claims, **characterised in that** one, two or three radicals from the group R², R³, R⁴, independently of one another, have a meaning as for formula la according to Claim 4 and any other radicals from the group R², R³, R⁴ denote H.

6. Process according to one of the preceding claims, **characterised in that** the acid used is a mixture of acetic acid with at least one strong acid.

7. Process according to Claim 6, **characterised in that** the strong acid used is hydrochloric acid, sulfuric acid, phosphoric acid and/or trifluoromethanesulfonic acid.

8. Process according to one of the preceding claims, **characterised in that** the starting-material solutions are combined in the flow reactor at a pressure greater than 1 bar.

9. Process according to one of the preceding claims, **characterised in that** the starting-material solutions are combined in the flow reactor at a temperature of 50°C to 180°C.

10. Process according to one of the preceding claims, **characterised in that** the flow reactor is held at a temperature above the boiling point of the reaction mixture, determined at atmospheric pressure.

11. Process according to one of the preceding claims, **characterised in that**, before step d) is carried out, the reaction mixture is fed out of the flow reactor into a downstream, temperature-controlled hold zone.

12. Process according to one of the preceding claims, **characterised in that** the volume flow rates of the starting-material solutions are selected in such a way that the residence time of the reaction mixture in the flow reactor and in any hold zone present is 0.1 to 600 seconds.

13. Process according to one of the preceding claims, **characterised in that**, before step d) is carried out, the reaction mixture is fed out of the flow reactor and any hold zone present into a temperature-controlled cooling zone in order to cool the reaction mixture.

## Revendications

1. Procédé de préparation d'alcènes par élimination d'eau à partir d'alcoolates ou d'alcools ayant au moins un groupement CH en position α par rapport à la fonction alcoolate ou alcool, **caractérisé en ce que**
a) l'alcoolate ou l'alcool est initialement introduit dans au moins un solvant et amené à température,
b) au moins un acide est initialement introduit séparément dans au moins un solvant et amené à température,
c) les solutions de produits de départ issues de a) et b) sont alimentées séparément les unes des autres dans au moins un réacteur continu thermostaté et fonctionnant en continu, qui comporte un ou plusieurs tubes de circulation, et y sont associées,
d) le mélange réactionnel est prélevé et récupéré, traité et/ou réagi davantage dans une ou plusieurs réactions en aval.

2. Procédé selon la revendication 1, **caractérisé en ce que** l'alcoolate ou l'alcool utilisé est un composé de formule I 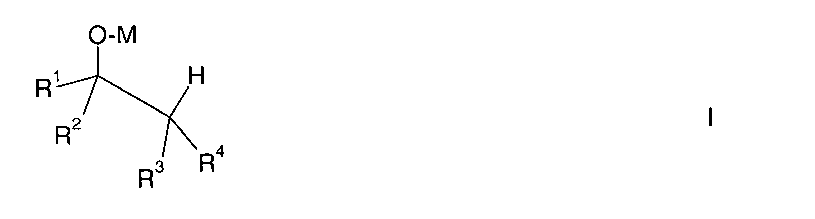 dans laquelle
M désigne un halogénure de métal alcalin, de métal alcalino-terreux, ou un atome de H et
R¹, R², R³, R⁴, indépendamment les uns des autres, désignent un radical aromatique ou aliphatique éventuellement substitué, pouvant contenir un ou plusieurs hétéro-atomes, où un, deux ou trois radicaux issus des groupements R¹ à R⁴ peuvent également désigner H, et où deux radicaux R¹, R², R³ et/ou R⁴, ou plus, peuvent être liés les uns aux autres.

3. Procédé selon la revendication 2, **caractérisé en ce que**
M désigne Li, MgCl, MgBr ou Mgl.

4. Procédé selon la revendication 2 ou 3, **caractérisé en ce que** R¹ a une signification identique à celle pour la formule la 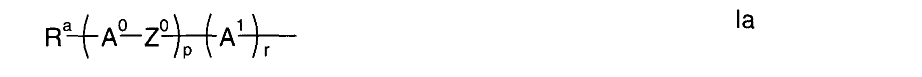 dans laquelle
R^{a} désigne un radical alkyle ayant de 1 à 15 atomes de C qui est non substitué, mono- ou polysubstitué par CN et/ou halogène, où, de plus, un ou plusieurs groupements CH₂ dans ces radicaux peuvent également être remplacés par -O-, -S-, -CH=CH-, -C≡C-, -OC-O- et/ou -O-CO- et/ou, de plus, un ou plusieurs groupements CH peuvent être remplacés par N ou P de telle sorte que deux atomes de O ne soient pas liés directement l'un à l'autre, ou, si r et/ou p sont différents de 0, également H, halogène, CN, SF₅ ou NCS,
A⁰, A¹ désignent chacun, indépendamment l'un de l'autre,
a) un radical 1,4-cyclohexénylène ou 1,4-cyclohexylène, dans lequel un ou deux groupements CH₂ non adjacents peuvent être remplacés par -O- ou -S-,
b) un radical 1,4-phénylène, dans lequel un ou deux groupements CH peuvent être remplacés par N,
c) un radical issu du groupe pipéridine-1,4-diyle, 1,4-bicyclo[2,2,2]octylène, phénanthrène-2,7-diyle, naphtalène-2,6-diyle, décahydronaphtalène-2,6-diyle et 1,2,3,4-tétrahydronaphtalène-2,6-diyle,
d) un radical divalent issu du groupe furane, pyrrole, thiophène, pyrazole, imidazole, 1,2-oxazole, 1,3-oxazole, thiazole, pyridine, pyridazine, pyrimidine, pyrazine, 2H-pyranne, 4H-pyranne, purine, ptéridine, 1 H-azépine, 3H-1,4-diazépine, indole, benzofurane, benzothiophène, quinoléine, isoquinoléine, phénazine, phénoxazine, phénothiazine et 1,4-benzodiazépine,
où les radicaux a), b), c) et d) peuvent être mono- ou poly-substitués par R^{a}, en particulier par halogène et/ou CN,
Z⁰ désigne -CO-O-, -O-CO-, -CF₂O-, -OCF₂-, -CH₂O-, -OCH₂-, -CH₂CH₂-, -(CH₂)₄-, -C₂F₄-, -CH₂CF₂-, -CF₂CH₂-, -CF=CF-, -CH=CH-, -C≡C- ou une liaison simple,
p vaut 0, 1, 2 ou 3 et
r vaut 0, 1 ou 2.

5. Procédé selon l'une parmi les revendications précédentes, **caractérisé en ce qu'**un, deux ou trois radicaux issus du groupe R², R³, R⁴, indépendamment les uns des autres, ont une signification identique à celle pour la formule la selon la revendication 4 et tout autre radical issu du groupe R², R³, R⁴ désigne H.

6. Procédé selon l'une parmi les revendications précédentes, **caractérisé en ce que** l'acide utilisé est un mélange d'acide acétique avec au moins un acide fort.

7. Procédé selon la revendication 6, **caractérisé en ce que** l'acide fort utilisé est l'acide chlorhydrique, l'acide sulfurique, l'acide phosphorique et/ou l'acide trifluorométhanesulfonique.

8. Procédé selon l'une parmi les revendications précédentes, **caractérisé en ce que** les solutions de produits de départ sont associées dans le réacteur continu à une pression supérieure à 1 bar.

9. Procédé selon l'une parmi les revendications précédentes, **caractérisé en ce que** les solutions de produits de départ sont associées dans le réacteur continu à une température allant de 50°C à 180°C.

10. Procédé selon l'une parmi les revendications précédentes, **caractérisé en ce que** le réacteur continu est maintenu à une température supérieure au point d'ébullition du mélange réactionnel, déterminé à la pression atmosphérique.

11. Procédé selon l'une parmi les revendications précédentes, **caractérisé en ce que**, avant la réalisation de l'étape d), le mélange réactionnel est alimenté hors du réacteur continu vers une zone de maintien en aval thermostatée.

12. Procédé selon l'une parmi les revendications précédentes, **caractérisé en ce que** les débits volumiques des solutions de produits de départ sont sélectionnés de telle sorte que le temps de séjour du mélange réactionnel dans le réacteur continu et dans une zone de maintien quelconque présente va de 0,1 à 600 secondes.

13. Procédé selon l'une parmi les revendications précédentes, **caractérisé en ce que**, avant la réalisation de l'étape d), le mélange réactionnel est alimenté hors du réacteur continu et de toute zone de maintien présente vers une zone de refroidissement thermostatée afin de refroidir le mélange réactionnel.
